# EUROPEAN PATENT SPECIFICATION

(11) **EP 1 303 238 B1**
(45) Date of publication and mention of the grant of the patent: **10.12.2003**
(21) Application number: 01961708.3
(22) Date of filing: 23.07.2001
(51) Int. Cl.: A61F 9/007

(54) **ULTRASOUND HANDPIECE**
ULTRASCHALLHANDSTÜCK
PIECE A MAIN A ULTRASONS

(30) Priority: 25.07.2000 US 624643
(43) Date of publication of application: 23.04.2003
(73) Proprietor: Alcon, Inc., 6331 Hünenberg (CH)
(72) Inventor: CHON, James, Y., Irvine, CA 92604 (US)
(74) Representative: Hanna, Peter William Derek
(86) International application number: US0123222
(87) International publication number: WO02007659

(56) References cited:
- WO-A-94/21183
- US-A- 4 819 635
- US-A- 4 981 756
- US-A- 5 505 693

## Description

This invention relates to ultrasonic devices and more particularly to an ophthalmic phacoemulsification handpiece.

### Background of the Invention

A typical ultrasonic surgical device suitable for ophthalmic procedures consists of an ultrasonically driven handpiece, an attached hollow cutting tip, an irrigating sleeve and an electronic control console. The handpiece assembly is attached to the control console by an electric cable and flexible tubings. Through the electric cable, the console varies the power level transmitted by the handpiece to the attached cutting tip and the flexible tubings supply irrigation fluid to and draw aspiration fluid from the eye through the handpiece assembly.

The operative part of the handpiece is a centrally located, hollow resonating bar or horn directly attached to a set of piezoelectric crystals. The crystals supply the required ultrasonic vibration needed to drive both the horn and the attached cutting tip during phacoemulsification and are controlled by the console. The crystal/horn assembly is suspended within the hollow body or shell of the handpiece at its nodal points by relatively inflexible mountings. The handpiece body terminates in a reduced diameter portion or nosecone at the body's distal end. The nosecone is externally threaded to accept the irrigation sleeve. Likewise, the horn bore is internally threaded at its distal end to receive the external threads of the cutting tip. The irrigation sleeve also has an internally threaded bore that is screwed onto the external threads of the nosecone. The cutting tip is adjusted so that the tip projects only a predetermined amount past the open end of the irrigating sleeve.

When used to perform phacoemulsification, the ends of the cutting tip and irrigating sleeve are inserted into a small incision of predetermined width in the cornea, sclera, or other location in the eye tissue in order to gain access to the anterior chamber of the eye. The cutting tip is ultrasonically vibrated along its longitudinal axis within the irrigating sleeve by the crystal-driven ultrasonic horn, thereby emulsifying upon contact the selected tissue in situ. The hollow bore of the cutting tip communicates with the bore in the horn that in turn communicates with the aspiration line from the handpiece to the console. A reduced pressure or vacuum source in the console draws or aspirates the emulsified tissue from the eye through the open end of the cutting tip, the bore of the cutting tip, the horn bore, and the aspiration line and into a collection device. The aspiration of emulsified tissue is aided by a saline flushing solution or irrigant that is injected into the surgical site through the small annular gap between the inside surface of the irrigating sleeve and the outside surface of the cutting tip.

There have been prior attempts to combine ultrasonic longitudinal motion of the cutting tip with rotational or oscillating motion of the tip, see U.S. Patent Nos. 5,222,959, 5,492,528, 5,827,292 (Anis), 5,722,945, 5,730,718, 5,911,699(Anis, et al.) and 4,504,264 (Kelman).

In particular US-A-5722945 discloses an ultrasonic surgical handpiece comprising an outer shell housing an ultrasonic horn in combination with a piezoelectric stack and connected to a motor drive shaft. The nature and placement of any seals and supports is not disclosed in this document.

US-A-4505264 discloses a device wherein seals support within a shell a vibrational hub arranged for longitudinal and separately excited rotational oscillation.

These prior attempts have used electric motors to provide the rotation of the tip which require O-ring seals that can fail, particularly under the extreme heat and pressure of autoclave sterilization, causing failure of the motor and/or the piezoelectric elements.

Accordingly, a need continues to exist for a reliable ultrasonic handpiece that combines both longitudinal and rotational motion.

### Brief Summary of the Invention

The present invention provides an ultrasound surgical handpiece in accordance with claims which follow.

The present invention improves upon prior art ultrasonic devices by providing a handpiece having a set of longitudinally vibrating piezoelectric elements and an electric motor to provide rotational or oscillatory movement to the ultrasound horn. The ultrasound horn and the shaft of the motor are held within the handpiece shell by spring-loaded seals. The portions of the ultrasound horn and the motor shaft that rotate and longitudinally vibrate within the seals are hardened, such as by plating with titanium nitride or other hard coating. Such a hardening process increases the life of the handpiece.

It is accordingly an object of the present invention to provide an ultrasound handpiece having both longitudinal and torsional motion.

It is a further object of the present invention to provide an ultrasound handpiece with a hardened horn.

Another objective of the present invention is to provide a more reliable ultrasound handpiece.

Other objects, features and advantages of the present invention will become apparent with reference to the drawings, and the following description of the drawings and claims.

### Brief Description of the Drawings

FIG. 1 is a perspective view of the ultrasonic handpiece of the present invention.
FIG. 2 is a cross-section view of the ultrasonic handpiece of the present invention.

### Detailed Description of the Invention

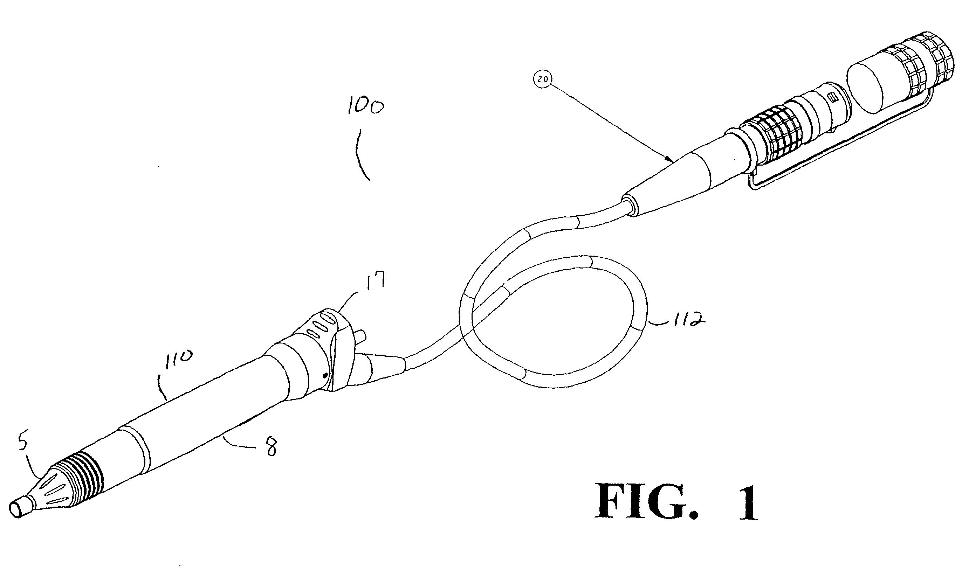

As best seen in FIG. 1, handpiece 100 of the present invention generally consists of handpiece body 110, electric cable 112 and electric connector 20.

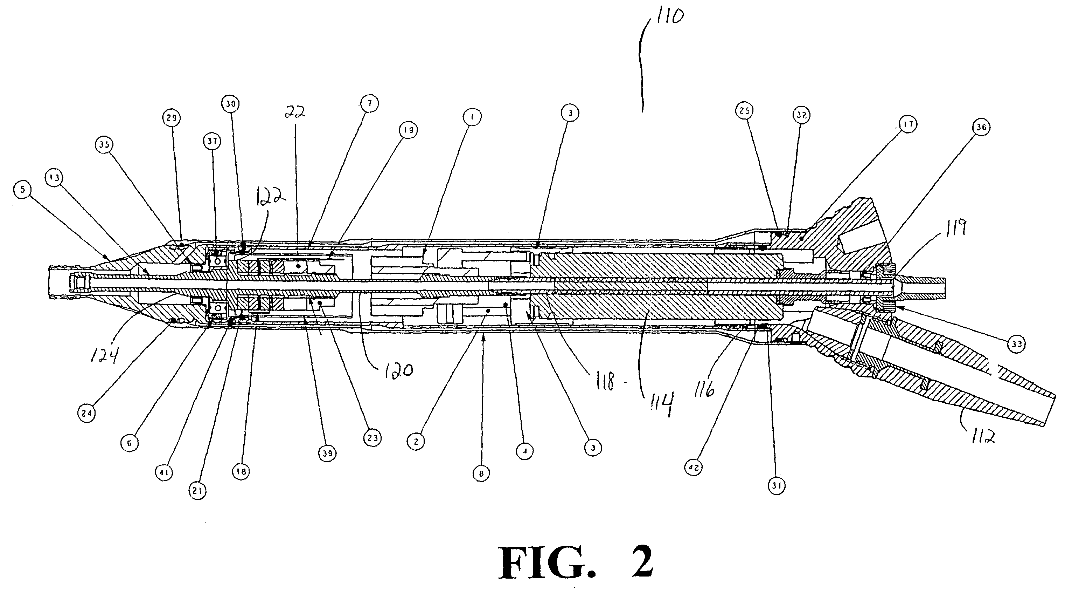

As best seen in FIG. 2, handpiece body 110 of the present invention generally includes nosecone shell 5, handpiece inner shell 7, handpiece outer shell 8 and handpiece end piece 17. Motor 114 is mounted within inner shell 7 by motor mount 3 and seal 116 and may contain devices, such as torsioner ring 2 and torsioner wire 4, for limiting the rotary movement of motor 114. Attached to distal end of shaft 118 of motor 114 is driveshaft 120 portion of horn 13. Piezoelectric stack 21 is received over driveshaft 120 portion of horn 13 and held in place against end plate 122 on driveshaft 120 by split nuts 22 and 23. Front bulkhead 1 is threaded onto driveshaft 120 behind nut 23 to de-couple the ultrasonic energy generated by piezoelectric stack 21 from motor 114. Power is supplied to piezoelectric stack by electrodes 18 and 19, and the entire assembly may be surrounded by insulating sleeve 39. Ultrasound horn 13 is threaded into endplate 122 on driveshaft 120. Horn 13 is held within inner shell 7 by spring-loaded seal 35 and by bearing 37 contacting hub 124 of horn 13. Piezoelectric stack 21 is supported by bearings 6 and 41. Inner shell 7 is held within outer shell 8 by silicone rubber or elastomeric O-rings 30, 31, 41 and 42. Nosecone shell 5 is received in the distal end of outer shell 8 and sealed fluid tight by silicone rubber or elastomeric O-rings 24 and 29. End piece 17 is received on the proximal end of outer shell 8 and sealed fluid tight by silicone rubber or elastomeric O-rings 25 and 32. Proximal end 119 of motor shaft 118 is held within end piece 17 by spring-loaded seal 36 and by spanner ring 33.

In use, motor 114 is energized and causes motor shaft 118 to rotate. Rotation of shaft 118 causes rotation of driveshaft 120, piezoelectric stack 21 and horn 13. Rotation of horn 13 and motor shaft 118 causes friction and wear at the interface between hub 124 and seal 35 and distal end 119 of shaft 118 and seal 36. This friction can cause excessive wear on hub 124 and shaft 118, which preferably are made from titanium, by seals 35 and 36, which are preferably carbon/graphite filled, a very abrasive material. When such wear occurs, handpiece body 110 may no longer be sealed fluid tight, particularly in a steam autoclave. To prevent excessive wear on hub 124 and shaft 118, hub 124 and proximal end 119 of shaft 118 may be plated or coated with any hard coating such as titanium nitride, zirconium nitride, chromium nitride or boron carbide (also know as black diamond), but titanium nitride is preferred. While titanium nitride coatings may be applied as thin as 2-4 microns, the inventors have found that such a thin coating is easily cracked when applied over a relatively soft material such as titanium. Therefore, it has been discovered that a titanium nitride coating greater than 4 microns, and preferably between 9-12 microns gives the best results. Coatings greater than 4 microns, however, may change the surface morphology of the coating, resulting in a sandpaper-like finish undesirable for a sealing surface. The inventors have discovered that is such situations, polishing of the surface, for example, with a diamond powdered paste, removing approximately 1 micron of material or less, results in a satisfactory surface.

While certain embodiments of the present invention have been described above, these descriptions are given for purposes of illustration and explanation. Variations, changes, modifications and departures from the systems and methods disclosed above may be adopted without departure from the scope of the present invention as defined by the following claims.

## Claims

1. An ultrasound surgical handpiece (110), comprising:
a) an outer shell (8);
b) an ultrasound horn (13) having a distal hub (124) and a proximal drive shaft (120), the hub being held within the shell by a first seal (35), the drive shaft receiving a piezoelectric stack (21) and being connected to a distal end of a motor shaft (118) with a proximal end (119) of the motor shaft held within the shell by a second seal (36), the first and second seals thereby locating the horn and the shaft within the outer shell; and
c) a hard coating on the hub (124).

2. The handpiece of claim 1, further comprising a hard coating on the proximal end (119) of the motor shaft (118).

3. The handpiece of claim 1 or claim 2, wherein the hard coating is a polished hard coating.

4. The handpiece of any one of claims 1 to 3, wherein the hard coating is titanium nitride.

5. The handpiece of any one of claims 1 to 4, wherein the coating is greater than 4 microns thick.

6. The handpiece of claim 5, wherein the coating is between approximately 9 microns and 12 microns thick.

## Patentansprüche

1. Chirurgisches Ultraschallhandstück (110), das folgendes umfaßt:
a) eine äußere Schale (8),
b) ein Ultraschallhom (13) mit einer distalen Nabe (124) und einer proximalen Antriebswelle (120), wobei die Nabe in der Schale mit einer ersten Dichtung (35) gehalten wird, die Antriebswelle einen piezoelektrischen Stapel (21) aufnimmt und mit einem distalen Ende einer Motorwelle (118) verbunden ist, wobei ein proximales Ende (119) der Motorwelle in der Schale mit einer zweiten Dichtung (36) gehalten wird, so daß die erste und die zweite Dichtung das Horn und die Welle in der äußeren Schale in Position halten, und
c) eine harte Beschichtung auf der Nabe (124).

2. Handstück nach Anspruch 1, das weiterhin eine harte Beschichtung auf dem proximalen Ende (119) der Motorwelle (118) umfaßt.

3. Handstück nach Anspruch 1 oder Anspruch 2, bei dem die harte Beschichtung eine polierte harte Beschichtung ist.

4. Handstück nach einem der Ansprüche 1 bis 3, bei dem die harte Beschichtung Titannitrit ist.

5. Handstück nach einem der Ansprüche 1 bis 4, bei dem die Beschichtung mehr als 4 Mikrometer dick ist.

6. Handstück nach Anspruch 5, bei dem die Beschichtung zwischen etwa 9 Mikrometer und 12 Mikrometer dick ist.

## Revendications

1. Pièce à main chirurgicale à ultrasons (110), comprenant :
a) un boîtier externe (8) ;
b) une trompe à ultrasons (13) possédant un manchon distal (124) et un arbre d'entraînement proximal (120), le manchon étant maintenu à l'intérieur du boîtier par un premier joint (35), l'arbre d'entraînement recevant un empilement piézoélectrique (21) et étant connecté à une extrémité distale d'un arbre moteur (118) avec une extrémité proximale (119) de l'arbre moteur maintenue à l'intérieur du boîtier par un second joint (36), le premier et le second joints positionnant la trompe et l'arbre à l'intérieur du boîtier externe ; et
c) une couche de revêtement dure sur le manchon (124).

2. Pièce à main selon la revendication 1, comprenant de plus une couche de revêtement dure sur l'extrémité proximale (119) de l'arbre moteur (118).

3. Pièce à main selon l'une des revendications 1 ou 2, dans laquelle la couche de revêtement dure est une couche de revêtement dure polie.

4. Pièce à main selon l'une des revendications 1 à 3, dans laquelle la couche de revêtement dure est en nitrure de titane.

5. Pièce à main selon l'une des revendications 1 à 4, dans laquelle la couche de revêtement dure présente une épaisseur supérieure à 4 microns.

6. Pièce à main selon la revendication 5, dans laquelle la couche de revêtement dure présente une épaisseur comprise approximativement entre 9 et 12 microns.
